(19) Europäisches Patentamt
European Patent Office
Office européen des brevets

(11) Veröffentlichungsnummer: **0 537 538 A1**

# (12) EUROPÄISCHE PATENTANMELDUNG

(21) Anmeldenummer: **92116537.9**

(51) Int. Cl.⁵: **C07C 59/68**

(22) Anmeldetag: **28.09.92**

(30) Priorität: **11.10.91 DE 4133674**

(43) Veröffentlichungstag der Anmeldung:
**21.04.93 Patentblatt 93/16**

(84) Benannte Vertragsstaaten:
**BE CH DE DK ES FR GB IT LI NL**

(71) Anmelder: **BAYER AG**

**W-5090 Leverkusen 1 Bayerwerk(DE)**

(72) Erfinder: **Förster, Heinz, Dr.**
**Am Eckbusch 47**
**W-5600 Wuppertal 1(DE)**
Erfinder: **Marhold, Albrecht, Dr.**
**Carl-Duisberg-Strasse 329**
**W-5090 Leverkusen(DE)**
Erfinder: **Santel, Hans-Joachim, Dr.**
**Grünstrasse 9a**
**W-5090 Leverkusen(DE)**
Erfinder: **Lürssen, Klaus, Dr.**
**August-Kierspel-Strasse 145**
**W-5060 Bergisch Gladbach 2(DE)**
Erfinder: **Schmidt, Robert R., Dr.**
**Im Waldwinkel 110**
**W-5060 Bergisch Gladbach 2(DE)**

## (54) Fluorierte Phenoxyphenoxycarbonsäurederivate als Herbizide.

(57) Beschrieben wurden neue fluorierte Phenoxyphenoxycarbonsäurederivate der Formel (I)

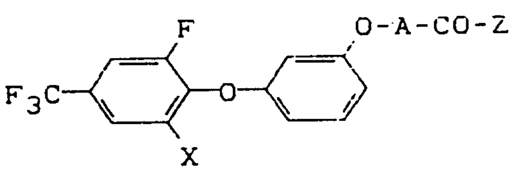

(I)

in welcher X, A und Z die in der Beschreibung angegebene Bedeutung haben, sowie mehrere Verfahren zu deren Herstellung.

Die neuen Phenoxyphenoxycarbonsäurederivate werden als Herbizide verwendet.

EP 0 537 538 A1

Die Erfindung betrifft neue fluorierte Phenoxyphenoxycarbonsäurederivate, Verfahren zu ihrer Herstellung und ihre Verwendung als Herbizide.

Es ist bekannt, daß bestimmte Phenoxypropionsäure-Derivate, wie z.B. α-(3-(2,6-Dichlor-4-trifluormethyl-phenoxy)-phenoxy)-propionsäure-methylester herbizid wirksam sind (vgl. DE-OS 2805981; vgl. auch DE-OS 2805982, DE-OS 3319290, DE-OS 3425123). Die Wirksamkeit dieser bekannten Verbindungen ist jedoch, insbesondere bei niedrigen Dosierungen, nicht immer ganz zufriedenstellend.

Es wurden nun neue fluorierte Phenoxyphenoxycarbonsäurederivate der allgemeinen Formel (I) gefunden,

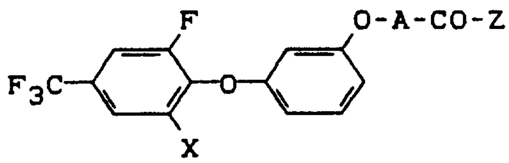

(I)

in welcher

A für Alkandiyl mit 1 bis 3 Kohlenstoffatomen steht,

X für Wasserstoff oder Halogen steht und

Z für Chlor, Hydroxy, Amino, $C_1$-$C_6$-Alkylamino, $C_3$-$C_4$-Alkenylamino, $C_3$-$C_4$-Alkinylamino, Phenylamino, Benzylamino, $C_1$-$C_4$-Alkoxycarbonyl-$C_1$-$C_2$-alkylamino, Cyanamino, Di-($C_1$-$C_4$-alkyl)-amino, Di-($C_3$-$C_4$-alkenyl)-amino, $C_1$-$C_4$-Alkylsulfonylamino, Phenylsulfonylamino, Tolylsulfonylamino, Hydroxyamino, $C_1$-$C_6$-Alkoxyamino, N-($C_1$-$C_6$-Alkoxy)-N-($C_1$-$C_4$-alkyl)-amino, Hydrazino, $C_1$-$C_4$-Alkylsulfonylhydrazino, Phenylsulfonylhydrazino, Tolylsulfonylhydrazino, $C_1$-$C_4$-Alkylthio, Phenylthio, Benzylthio, $C_1$-$C_4$-Alkoxycarbonyl-$C_1$-$C_2$-alkylthio oder für die Gruppierung -O-$R^1$ steht, worin

$R^1$ für einen gegebenenfalls durch Fluor und/oder Chlor substituierten Rest aus der Reihe $C_1$-$C_6$-Alkyl, $C_3$-$C_4$-Alkenyl, $C_3$-$C_4$-Alkinyl, $C_3$-$C_6$-Cycloalkyl, $C_1$-$C_4$-Alkoxy-$C_1$-$C_4$-alkyl, $C_1$-$C_4$-Alkoxy-$C_1$-$C_4$-Alkoxy-$C_1$-$C_4$-alkyl, $C_1$-$C_4$-Alkylthio-$C_1$-$C_4$-alkyl, $C_1$-$C_4$-Alkylsulfinyl-$C_1$-$C_4$-alkyl, $C_1$-$C_4$-Alkylsulfonyl-$C_1$-$C_4$-alkyl, Phenoxy-$C_1$-$C_3$-alkyl, Trimethylsilylmethyl, Phenylthio-$C_1$-$C_3$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-(Di)-Alkylamino-carbonyl-$C_1$-$C_2$-alkyl, Benzyl, Pyrazolyl-$C_1$-$C_4$-alkyl, $C_2$-$C_4$-Alkylidenamino oder für ein Ammonium-, ein $C_1$-$C_4$-Alkylammonium, ein Natrium-, Kalium- oder Calcium-äquivalent steht, oder die Gruppierung

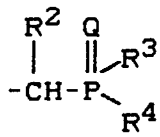

steht, worin

$R^2$ für Wasserstoff, $C_1$-$C_4$-Alkyl, Phenyl, Furyl, Thienyl oder Pyridyl steht,

$R^3$ für $C_1$-$C_4$-Alkyl oder $C_1$-$C_4$-Alkoxy steht,

$R^4$ für $C_1$-$C_4$-Alkoxy steht und

Q für Sauerstoff oder Schwefel steht, oder

$R^1$ für die Gruppierung $-(CH_2)_n-R^5$ steht, worin

n für die Zahlen 0, 1 oder 2 steht und

$R^5$ für einen gegebenenfalls durch Fluor, Chlor, Brom und/oder $C_1$-$C_4$-Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Isoxazolidinyl, Pyridinyl oder Pyrimidinyl steht.

Die Verbindungen der Formel (I) können (bei verzweigtem A = Alkandiyl) ein asymmetrisch substituiertes Kohlenstoffatom enthalten und können deshalb in verschiedenen enantiomeren Formen vorliegen. Die Erfindung betrifft sowohl die möglichen einzelnen Isomeren als auch die Gemische dieser Isomeren.

Man erhält die neuen fluorierten Phenoxyphenoxycarbonsäurederivate der Formel (I), wenn man

(a) fluorierte Phenoxyphenole der allgemeinen Formel (II)

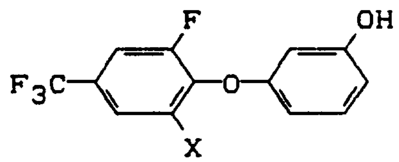

(II)

in welcher
X die oben angegebene Bedeutung hat,
mit Carbonsäurederivaten der allgemeinen Formel (III)

Y-A-CO-Z (III)

in welcher
A und Z die oben angegebene Bedeutung haben und
Y für eine nucleophile Abgangsgruppe steht,
gegebenenfalls in Gegenwart eines Säureakzeptors und gegebenenfalls in Gegenwart eines Verdünnungsmittels umsetzt, oder wenn man
(b) Fluorbenzolderivate der allgemeinen Formel (IV)

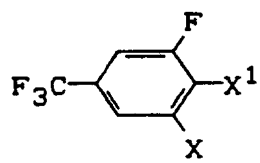

(IV)

in welcher
X die oben angegebene Bedeutung hat und
$X^1$ für Halogen steht,
mit Hydroxyphenoxycarbonsäurederivaten der allgemeinen Formel (V)

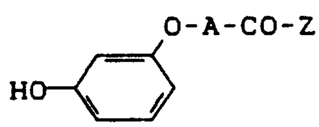

(V)

in welcher
A und Z die oben angegebene Bedeutung haben,
gegebenenfalls in Gegenwart eines Säureakzeptors und gegebenenfalls in Gegenwart eines Verdünnungsmittels umsetzt, oder wenn man
(c) für den Fall, daß Z für Hydroxy steht und A und X die oben angegebene Bedeutung haben,
Verbindungen der Formel (I), in welcher Z für Methoxy oder Ethoxy steht und A und X die oben angegebene Bedeutung haben,
mit einem Alkalihydroxid in Gegenwart von Wasser und gegebenenfalls in Gegenwart eines organischen Lösungsmittels umsetzt und dann - gegebenenfalls nach Einengen - mit einer Mineralsäure ansäuert, oder wenn man
(d) für den Fall, daß Z für Chlor steht und A und X die oben angegebene Bedeutung haben
Verbindungen der Formel (I), in welcher Z für Hydroxy steht und A und X die oben angegebene Bedeutung haben,
mit einem Chlorierungsmittel gegebenenfalls in Gegenwart eines Katalysators und gegebenenfalls in Gegenwart eines Verdünnungsmittels umsetzt, oder wenn man
(e) für den Fall, daß Z mit Ausnahme von Chlor die oben angegebene Bedeutung hat und A und X die oben angegebene Bedeutung haben,
Verbindungen der Formel (I), in welcher Z für Chlor steht und A und X die oben angegebene Bedeutung haben, mit Verbindungen der allgemeinen Formel (VI)

H-Z (VI)

in welcher

Z mit Ausnahme von Chlor die oben angegebene Bedeutung hat,

gegebenenfalls in Gegenwart eines Säureakzeptors und gegebenenfalls in Gegenwart eines Verdünnungsmittels umsetzt, oder wenn man

(f) für den Fall, daß Z für die Gruppierung $-O-R^1$ steht, worin $R^1$ mit Ausnahme von Ammonium, Alkylammonium, Alkalimetall und Erdalkalimetall die oben angegebene Bedeutung hat und A und X die oben angegebene Bedeutung haben,

Verbindungen der Formel (I), in welcher Z für Hydroxy, Methoxy oder Ethoxy steht und A und X die oben angegebene Bedeutung haben, mit Hydroxyverbindungen der allgemeinen Formel (VII)

$HO-R^1$ (VII)

in welcher

$R^1$ mit Ausnahme von Ammonium, Alkylammonium, Alkalimetall und Erdalkalimetall die oben angegebene Bedeutung hat,

gegebenenfalls in Gegenwart eines Katalysators und gegebenenfalls in Gegenwart eines Verdünnungsmittels umsetzt.

Die neuen fluorierten Phenoxyphenoxycarbonsäurederivate der allgemeinen Formel (I) zeichnen sich durch starke herbizide Wirksamkeit aus.

Überraschenderweise zeigen die erfindungsgemäßen Verbindungen der Formel (I) erheblich stärkere Wirkung gegen bestimmte Problemunkräuter als die bekannte Verbindung α-(3-(2,6-Dichlor-4-trifluormethyl-phenoxy)-phenoxy)-propionsäure-methylester, welche ein strukturell ähnlicher vorbekannter Wirkstoff gleicher Wirkungsrichtung ist.

Die Erfindung betrifft vorzugsweise Verbindungen der Formel (I), in welcher

A für Methan-1,1-diyl (Methylen, $-CH_2-$), Ethan-1,1-diyl (Ethyliden,

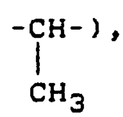

Ethan-1,2-diyl (Ethylen, Dimethylen, $-CH_2CH_2-$), Propan-1,1-diyl (Propyliden,

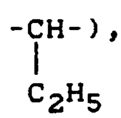

X für Wasserstoff, Fluor oder Chlor steht und

Z für Chlor, Hydroxy, Amino, $C_1$-$C_4$-Alkylamino, Phenylamino, $C_1$-$C_4$-Alkoxy-carbonyl-$C_1$-$C_2$-alkylamino, Di-($C_1$-$C_3$-alkyl)-amino, $C_1$-$C_4$-Alkylsulfonylamino, Phenylsulfonylamino, Hydroxyamino, Cyanamino, $C_1$-$C_4$-Alkoxyamino, N-($C_1$-$C_4$-Alkoxy)-N-($C_1$-$C_3$-alkyl)-amino, Hydrazino, $C_1$-$C_4$-Alkylsulfonylhydrazino, Phenylsulfonylhydrazino, $C_1$-$C_4$-Alkylthio oder $C_1$-$C_4$-Alkoxy-carbonyl-$C_1$-$C_2$-alkylthio oder für die Gruppierung $-O-R^1$ steht, worin

$R^1$ für $C_1$-$C_4$-Alkyl, $C_1$-$C_2$-Alkoxy-$C_1$-$C_2$-alkyl, $C_1$-$C_2$-Alkoxy-$C_1$-$C_2$-alkoxy-$C_1$-$C_2$-alkyl, $C_1$-$C_2$-Alkylthio-$C_1$-$C_2$-alkyl, $C_1$-$C_2$-Alkylsulfinyl-$C_1$-$C_2$-alkyl, $C_1$-$C_2$-Alkylsulfonyl-$C_1$-$C_2$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-(Di)-Alkylamino-carbonyl-$C_1$-$C_2$-alkyl, Benzyl, Trimethylsilylmethyl oder für ein Ammonium-, $C_1$-$C_3$-Alkylammonium-, Natrium-, oder Kalium-äquivalent steht, oder für die Gruppierung

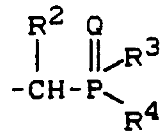

steht, worin

- $R^2$ für Wasserstoff, Methyl, Phenyl, Furyl, Thienyl oder Pyridyl steht,
- $R^3$ für Methoxy oder Ethoxy steht,
- $R^4$ für Methoxy oder Ethoxy steht und
- Q für Sauerstoff oder Schwefel steht, oder
- $R^1$ für die Gruppierung $-(CH_2)_n-R^5$ steht, worin
- n für die Zahlen 0, 1 oder 2 steht und
- $R^5$ für einen gegebenenfalls durch Chlor und/oder Methyl substituierten heterocyclischen Rest aus der Reihe Furyl, Tetrahydrofuryl, Thienyl, Perhydropyranyl, Oxazolyl, Thiazolyl, Isoxazolidinyl und Dioxolanyl steht.

Die Erfindung betrifft insbesondere Verbindungen der Formel (I), in welcher

- A für Ethan-1,1-diyl

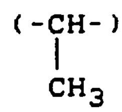

steht,

- X für Wasserstoff, Fluor oder Chlor steht und
- Z die oben als bevorzugt angegebene Bedeutung hat.

Die R-Isomeren der Verbindungen der Formel (I) werden ganz besonders bevorzugt.

Verwendet man für das erfindungsgemäße Verfahren (a) beispielsweise 3-(2-Fluor-4-trifluormethyl-phenoxy)-phenol und $\alpha$-Brom-propionsäure-methylester als Ausgangsstoffe, so kann der Reaktionsablauf durch das folgende Formelschema wiedergegeben werden:

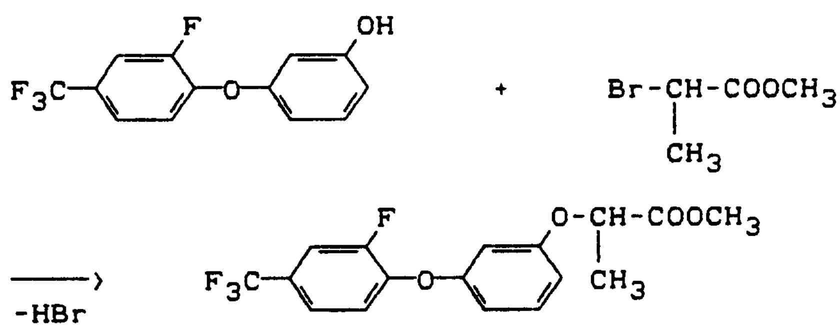


Verwendet man für das erfindungsgemäße Verfahren (b) beispielsweise 3,4,5-Trifluor-benzotrifluorid und $\alpha$-(3-Hydroxy-phenoxy)-propionsäure-propylester als Ausgangsstoffe, so kann der Reaktionsablauf durch das folgende Formelschema wiedergegeben werden:

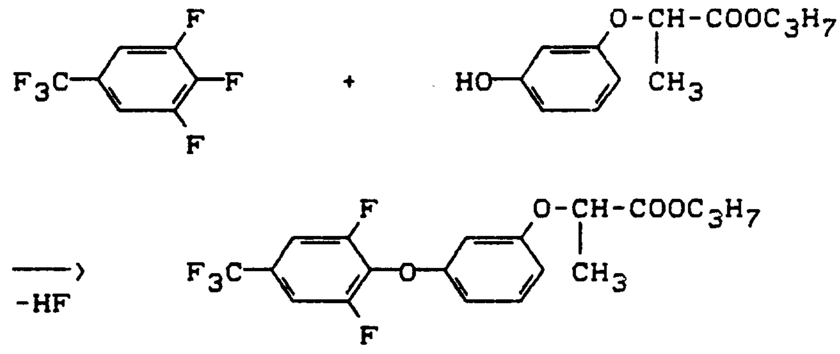


Verwendet man für das erfindungsgemäße Verfahren (c) beispielsweise α-(3-(2-Chlor-6-fluor-4-trifluormethyl-phenoxy)-phenoxy)-propionsäure-ethylester und Natronlauge als Ausgangsstoffe, so kann der Reaktionsablauf durch das folgende Formelschema wiedergegeben werden:

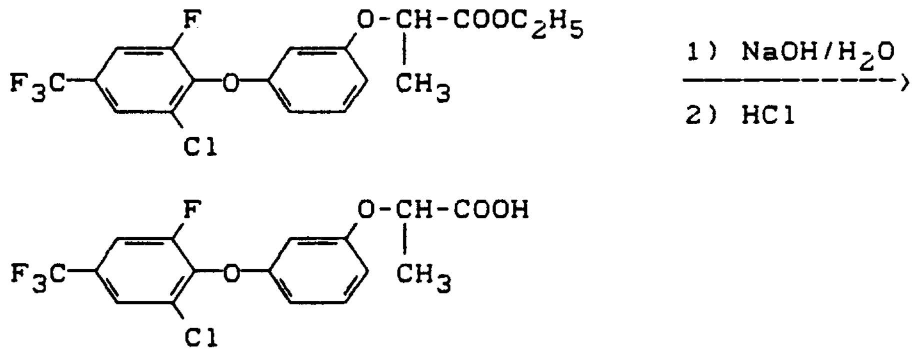


Verwendet man für das erfindungsgemäße Verfahren (d) beispielsweise α-(3-(2-Fluor-4-trifluormethyl-phenoxy)-phenoxy)-propionsäure und Thionylchlorid als Ausgangsstoffe, so kann der Reaktionsablauf durch das folgende Formelschema wiedergegeben werden:

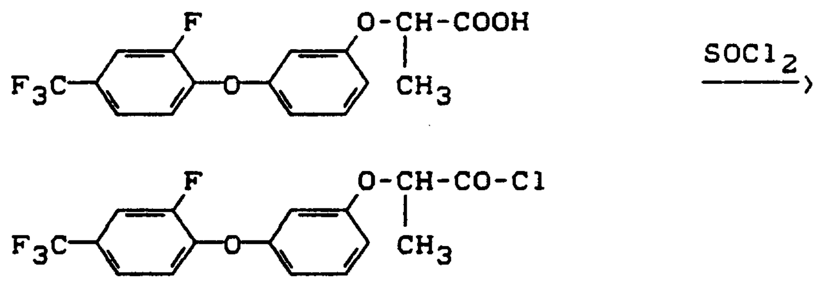


Verwendet man für das erfindungsgemäße Verfahren (e) beispielsweise α-(3-(2,6-Difluor-4-trifluormethyl-phenoxy)-phenoxy)-propionsäurechlorid und Butanol als Ausgangsstoffe, so kann der Reaktionsablauf durch das folgende Formelschema wiedergegeben werden:

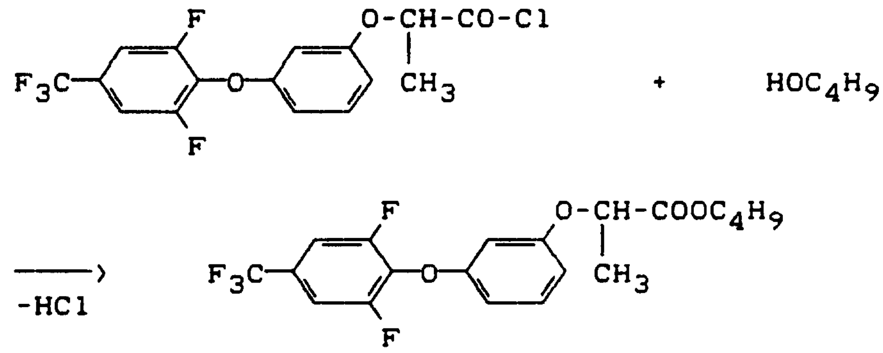


Verwendet man für das erfindungsgemäße Verfahren (f) beispielsweise α-(2-Chlor-6-fluor-4-trifluormethyl-phenoxy)-phenoxy)-propionsäure und Isopropanol als Ausgangsstoffe, so kann der Reaktionsablauf durch das folgende Formelschema wiedergegeben werden:

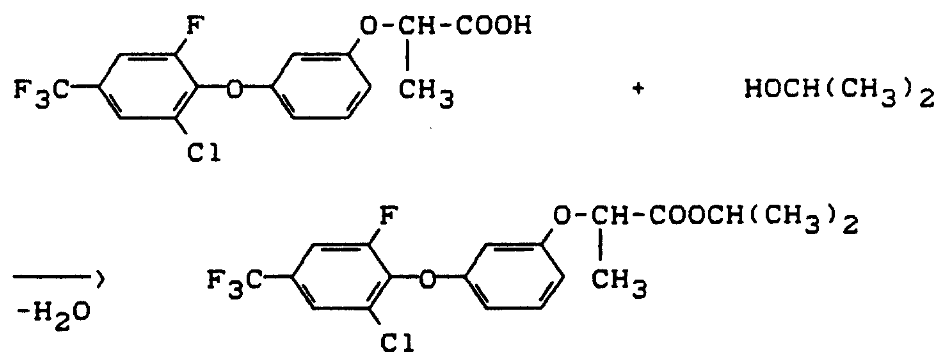


Die beim erfindungsgemäßen Verfahren (a) zur Herstellung von Verbindungen der Formel (I) als Ausgangsstoffe zu verwendenden fluorierten Phenoxyphenole sind durch die Formel (II) allgemein definiert.

In Formel (II) hat X vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für X angegeben wurde.

Als Beispiele für die Ausgangsstoffe der Formel (II) seien genannt:
3-(2-Fluor-4-trifluormethyl-phenoxy)-phenol, 3-(2,6-Difluor-4-trifluormethyl-phenoxy)-phenol und 3-(2-Chlor-6-fluor-4-trifluormethyl-phenoxy)-phenol.

Die Ausgangsstoffe der Formel (II) sind bekannt und/oder können nach an sich bekannten Verfahren hergestellt werden (vgl. DE-OS 2805981, EP-A 370644).

Die beim erfindungsgemäßen Verfahren (a) weiter als Ausgangsstoffe zu verwendenden Carbonsäurederivate sind durch die Formel (III) allgemein definiert. In Formel (III) haben A und Z vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt angegeben wurde und Y steht vorzugsweise für Chlor, Brom, Iod, gegebenenfalls durch Fluor oder Chlor substituiertes $C_1$-$C_4$-Alkylsulfonyloxy oder gegebenenfalls durch Fluor, Chlor, Brom oder Methyl substituiertes Phenylsulfonyloxy, insbesondere für Chlor, Brom, Methylsulfonyloxy, Phenylsulfonyloxy oder 4-Methyl-phenylsulfonyloxy.

Als Beispiele für die Verbindungen der Formel (III) seien genannt:
α-Chlor-, α-Brom- und α-Iod-propionsäure-methylester, -ethylester, -propylester, -isopropylester, -butylester, -isobutylester und -sec-butylester, α-Methylsulfonyloxy-, α-Ethylsulfonyloxy-, α-Propylsulfonyloxy-, α-Butylsulfonyloxy-, α-Trifluormethylsulfonyloxy-, α-Phenylsulfonyloxy- und α-(4-Methyl-phenylsulfonyloxy)-propionsäure-methylester, -ethylester, -propylester, -butylester, -isopropylester, -isobutylester und -sec-butylester.

Unter den genannten Verbindungen der Formel (III) sind jeweils die R-Isomeren, die S-Isomeren und die racemischen Gemische dieser Isomeren zu verstehen.

Die Ausgangsstoffe der Formel (III) sind bekannt und/oder können nach an sich bekannten Verfahren hergestellt werden (vgl. DE-OS 27 58 002, DE-OS 28 54 542).

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren (a) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (a) in einem größeren Bereich variiert werden. Im allgemeinen arbeitet man bei Temperaturen zwischen 0 °C und 150 °C, vorzugsweise bei Temperaturen zwischen 20 °C und 100 °C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (a) jeweils nach üblichen Methoden (vgl. die Herstellungsbeispiele).

Die beim erfindungsgemäßen Verfahren (b) zur Herstellung von Verbindungen der Formel (I) als Ausgangsstoffe zu verwendenden Fluorbenzolderivate sind durch die Formel (IV) allgemein definiert.

In Formel (IV) hat X vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für X angegeben wurde; $X^1$ steht vorzugsweise für Fluor oder Chlor.

Als Beispiele für die Ausgangsstoffe der Formel (IV) seien genannt:
3,4-Difluor-benzotrifluorid, 3,4,5-Trifluor-benzotrifluorid und 3-Chlor-4,5-difluor-benzotrifluorid.

Die Ausgangsstoffe der Formel (IV) sind bekannt und/oder können nach an sich bekannten Verfahren hergestellt werden (vgl. EP-A 302326; EP-A 34402; DE-OS 3737986; EP-A 289036).

Die beim erfindungsgemäßen Verfahren (b) weiter als Ausgangsstoffe zu verwendenden Hydroxyphenoxycarbonsäurederivate sind durch die Formel (V) allgemein definiert. In Formel (V) haben A und Z vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt angegeben wurden.

Als Beispiele für die Ausgangsstoffe der Formel (V) seien genannt:
α-(3-Hydroxy-phenoxy)-propionsäure-methylester, -ethylester, -propylester, -isopropylester, -butylester, -isobutylester und -sec-butylester.

Die Ausgangsstoffe der Formel (V) sind bekannt und/oder können nach an sich bekannten Verfahren hergestellt werden (vgl. DE-OS 3150233).

Verfahren (b) wird vorzugsweise unter Verwendung eines Verdünnungsmittels durchgeführt. Es kommen vor allem diejenigen Verdünnungsmittel in Betracht, die bereits bei der Beschreibung des erfindungsgemäßen Verfahrens (a) genannt wurden. Aprotisch polare organische Lösungsmittel, wie z. B. Aceton, Acetonitril, Methylethylketon, Propionitril, Dimethylformamid, Dimethylacetamid, Dimethylsulfoxid, Sulfolan und N-Methylpyrrolidon werden besonders bevorzugt.

Verfahren (b) wird vorzugsweise in Gegenwart eines Säureakzeptors duchgeführt. Es kommen vor allem diejenigen Säureakzeptoren in Betracht, die bereits bei der Beschreibung des erfindungsgemäßen Verfahrens (a) genannt wurden.

Die Reaktionstemperaturen können bei dem erfindungsgemäßen Verfahren (b) in einem größeren Bereich variiert werden. Im allgemeinen arbeitet man bei Temperaturen zwischen 0 °C und 200 °C,

vorzugsweise bei Temperaturen zwischen 20 °C und 150 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (b) jeweils nach üblichen Methoden.

Die beim erfindungsgemäßen Verfahren (c) als Ausgangsstoffe zu verwendenden Verbindungen sind durch die Formel (I) mit der Maßgabe, daß Z für Methoxy oder Ethoxy steht, allgemein definiert. In diesem Fall haben A und X vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt angegeben wurden.

Als Beispiele für die Ausgangsstoffe zu Verfahren (c) seien genannt:
α-(3-(2-Fluor-4-trifluormethyl-phenoxy)-phenoxy)-propionsäure-methylester und -ethylester, α-(3-(2,6-Difluor-4-trifluormethyl-phenoxy)-phenoxy)-propionsäure-methyl ester und -ethylester sowie α-(3-(2-Chlor-6-fluor-4-trifluormethyl-phenoxy)-phenoxy)-propionsäure-methylester und -ethylester.

Die oben beschriebenen Ausgangsstoffe der Formel (I) für Verfahren (c) sind erfindungsgemäße neue Verbindungen; sie können nach dem erfindungsgemäßen Verfahren (a) oder (b) hergestellt werden.

Verfahren (c) wird unter Verwendung von Alkalihydroxiden durchgeführt. Als Beispiel hierfür seien Lithiumhydroxid, Natriumhydroxid und Kaliumhydroxid genannt. Vorzugsweise wird Natriumhydroxid verwendet.

Verfahren (c) wird in Gegenwart von Wasser und gegebenenfalls in Gegenwart eines organischen Lösungsmittels durchgeführt. Als organische Lösungsmittel werden vorzugsweise Alkohole, wie z. B. Methanol oder Ethanol eingesetzt.

Zum Ansäuern werden bei Verfahren (c) die üblichen Mineralsäuren, wie z. B. Salzsäure oder Schwefelsäure, verwendet.

Die Reaktionstemperaturen können bei dem erfindungsgemäßen Verfahren (c) in einem größeren Bereich variiert werden. Im allgemeinen arbeitet man bei Temperaturen zwischen 10 °C und 150 °C, vorzugsweise bei Temperaturen zwischen 20 °C und 100 °C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung von Verfahren (c) werden je Mol Ausgangsverbindung der Formel (I) im allgemeinen zwischen 0,1 und 10 Mol, vorzugsweise zwischen 0,5 und 2 Mol, Alkalihydroxid eingesetzt. Die Reaktionskomponenten werden im allgemeinen bei Raumtemperatur zusammengegeben und das Reaktionsgemisch wird gegegenenfalls bei erhöhter Temperatur, bis zum Reaktionsende gerührt. Die Aufarbeitung erfolgt nach üblichen Methoden (vgl. die Herstellungsbeispiele).

Die beim erfindungsgemäßen Verfahren (d) als Ausgangsstoffe zu verwendenden Verbindungen sind durch die Formel (I) mit der Maßgabe, daß Z für Hydroxy steht, allgemein definiert. In diesem Fall haben A und X vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt angegeben wurden.

Als Beispiele für die Ausgangsstoffe zu Verfahren (d) seien genannt:
α-(3-(2-Fluor-4-trifluormethyl-phenoxy)-phenoxy)-propionsäure, α-(3-(2,6-Difluor-4-trifluormethyl-phenoxy)-phenoxy)-propionsäure und α-(3-(2-Chlor-6-fluor-4-trifluormethyl-phenoxy)-phenoxy)-propionsäure.

Die oben beschriebenen Ausgangsstoffe der Formel (I) für Verfahren (d) sind erfindungsgemäße, neue Verbindungen; sie können nach dem erfindungsgemäßen Verfahren (c) hergestellt werden.

Verfahren (d) wird unter Verwendung eines Chlorierungsmittels durchgeführt. Es können die üblichen Mittel für die Umsetzung von Carbonsäuren zur Carbonsäurechloriden eingesetzt werden. Als Beispiele hierfür seien Phosgen, Thionylchlorid, Phosphorylchlorid und Benzotrichlorid genannt. Vorzugsweise wird Thionylchlorid als Chlorierungsmittel verwendet.

Verfahren (d) wird gegebenenfalls in Gegenwart eines Katalysators duchgeführt. Es können die für die Herstellung von Säurechloriden aus Säuren üblichen Katalysatoren, wie z. B. Pyridin oder Dimethylformamid verwendet werden.

Verfahren (d) wird gegebenenfalls in Gegenwart eines Verdünnungsmittels durchgeführt. Vorzugsweise kommen inerte organische Lösungsmittel aus der Reihe der halogenierten Kohlenwasserstoffe, wie z. B. Methylenchlorid, Chloroform, Tetrachlormethan oder 1,2-Dichlorethan, in Betracht.

Die Reaktionstemperaturen können bei dem erfindungsgemäßen Verfahren (d) in einem größeren Bereich variiert werden. Im allgemeinen arbeitet man bei Temperaturen zwischen 0 °C und 100 °C, vorzugsweise bei Temperaturen zwischen 10 °C und 90 °C.

Verfahren (d) wird im allgemeinen bei Normaldruck durchgeführt.

Zur Durchführung von Verfahren (d) werden je Mol Ausgangsverbindung der Formel (I) im allgemeinen zwischen 1 und 100 Mol, vorzugsweise zwischen 1,2 und 50 Mol, Chlorierungsmittel eingesetzt. Die Reaktionskomponenten werden im allgemeinen bei Raumtemperatur zusammengegeben und das Reaktionsgemisch wird, gegebenenfalls bei erhöhter Temperatur bis zum Ende der Umsetzung gerührt. Das nach Abdestillieren der flüchtigen Komponenten unter vermindertem Druck verbleibende Reaktionsprodukt kann durch Umkristallisation gereinigt werden, aber auch ohne weitere Reinigung für Folgeumsetzungen eingesetzt werden.

Die beim erfindungsgemäßen Verfahren (e) als Ausgangsstoffe zu verwendenden Verbindungen sind durch die Formel (I) mit der Maßgabe, daß Z für Chlor steht allgemein definiert. In diesem Fall haben A und X vorzugsweise bzw. insbesondere diejenigen Bedeutungen, die bereits oben im Rahmen der Beschreibung der erfindungsgemäßen Verbindungen der Formel (I) vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt genannt wurden.

Als Beispiele für die Ausgangsstoffe zu Verfahren (e) seien genannt:
α-(3-(2-Fluor-4-trifluormethyl-phenoxy)-phenoxy)-propionsäurechlorid, α-(3-(2,6-Difluor-4-trifluormethyl-phenoxy)-phenoxy)-propionsäurechlorid und α-(3-(2-Chlor-6-fluor-4-trifluormethyl-phenoxy)-phenoxy)-propionsäurechlorid.

Die oben beschriebenen Ausgangsstoffe der Formel (I) für Verfahren (e) sind erfindungsgemäße, neue Verbindungen; sie können nach dem erfindungsgemäßen Verfahren (d) hergestellt werden.

Die beim erfindungsgemäßen Verfahren (e) weiter als Ausgangsstoffe einzusetzenden Verbindungen sind durch die Formel (VI) allgemein definiert. In Formel (VI) hat Z vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt angegeben wurde.

Als Beispiele für die Ausgangsstoffe der Formel (VI) seien genannt:
Methylamin, Ethylamin, Propylamin, Isopropylamin, Anilin, Cyanamid, Dimethylamin, Diethylamin, Hydroxylamin, O-Methylhydroxylamin, Hydrazin, Methylsulfonylhydrazin, Methanol, Ethanol, Propanol, Isopropanol, Butanol, Iso-butanol, sec-Butanol, 2-Methoxy-ethanol, 2-Ethoxy-ethanol, 2-Methylthio-ethanol, 2-Ethylthio-ethanol, 2-Benzyloxy-ethanol, 3-Benzyloxy-propanol, 2-Benzylthio-ethanol, Hydroxymethanphosphonsäure-diethylester und -dimethylester, 1-Hydroxy-ethan-phosphonsäure-dimethylester und -diethylester, 1-Hydroxy-1-phenyl-methanphosphonsäure-dimethylester und -diethylester, Acetonoxim, 3-Hydroxyfuran, Furfurylalkohol, Perhydrofurfurylalkohol, Milchsäure-methylester und -ethylester und Glycolsäuremethylester und -ethylester.

Diese Verbindungen sind bekannte Synthesechemikalien.

Verfahren (e) wird vorzugsweise unter Verwendung eines Verdünnungsmittels durchgeführt. Es kommen vor allem diejenigen Verdünnungsmittel in Betracht, die bereits bei der Beschreibung des erfindungsgemäßen Verfahrens (a) genannt wurden.

Verfahren (e) wird vorzugsweise in Gegenwart eines Säureakzeptors durchgeführt. Es kommen vor allem diejenigen Säureakzeptoren in Betracht, die bereits bei der Beschreibung des erfindungsgemäßen Verfahrens (a) genannt wurden.

Die Reaktionstemperaturen können bei dem erfindungsgemäßen Verfahren (e) in einem größeren Bereich variiert werden. Im allgemeinen arbeitet man bei Temperaturen zwischen -20 °C und +100 °C, vorzugsweise bei Temperaturen zwischen 0 °C und 50 °C.

Das erfindungsgemäße Verfahren (e) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (e) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Umsetzungen werden im allgemeinen in einem geeigneten Verdünnungsmittel in Gegenwart eines Säureakzeptors durchgeführt und das Reaktionsgemisch wird mehrere Stunden bei der jeweils erforderlichen Temperatur gerührt.

Die Aufarbeitung erfolgt bei dem erfindungsgemäßen Verfahren (e) jeweils nach üblichen Methoden.

Die beim erfindungsgemäßen Verfahren (f) als Ausgangsstoffe zu verwendenden Verbindungen sind durch die Formel (I) mit der Maßgabe, daß Z für Hydroxy, Methoxy oder Ethoxy steht, allgemein definiert. In diesem Fall haben A und X vorzugsweise bzw. insbesondere diejenigen Bedeutungen, die bereits oben im Rahmen der Beschreibung der erfindungsgemäßen Verbindungen der Formel (I) vorzugsweise bzw. als

insbesondere bevorzugt angegeben wurden.

Als Beispiele für die Ausgangsstoffe der Formel (I) zu Verfahren (f) seien genannt: α-(3-(2-Fluor-4-trifluormethyl-phenoxy)-phenoxy)-propionsäure, α-(3-(2,6-Difluor-4-trifluormethyl-phenoxy)-phenoxy)-propionsäure und α-(3-(2-Chlor-6-fluor-4-trifluormethyl-phenoxy)-phenoxy)-propionsäure sowie jeweils auch die entsprechenden Methylester und Ethylester.

Die oben beschriebenen Ausgangsstoffe der Formel (I) für Verfahren (f) sind erfindungsgemäße, neue Verbindungen; sie können nach den erfindungsgemäßen Verfahren (a), (b) oder (c) hergestellt werden.

Die beim erfindungsgemäßen Verfahren (f) weiter als Ausgangsstoffe einzusetzenden Verbindungen sind durch die Formel (VII) allgemein definiert. In Formel (VII) steht vorzugsweise $R^1$ für $C_1$-$C_6$-Alkyl, $C_3$-$C_4$-Alkenyl, $C_3$-$C_4$-Alkinyl, $C_1$-$C_4$-Alkoxy-$C_1$-$C_4$-alkyl, $C_1$-$C_4$-Alkylthio-$C_1$-$C_4$-alkyl, Phenoxy-$C_1$-$C_3$-alkyl, Trimethylsilylmethyl, Phenylthio-$C_1$-$C_3$-alkyl, Benzyloxy-$C_1$-$C_3$-alkyl, Benzylthio-$C_1$-$C_3$-alkyl oder $C_1$-$C_4$-Alkoxy-carbonyl-$C_1$-$C_2$-alkyl.

Die Ausgangstoffe der Formel (VII) sind bekannte Synthesechemikalien.

Verfahren (f) wird vorzugsweise unter Verwendung eines Vedünnungsmittels durchgeführt. Als solche kommen insbesondere die Hydroxyverbindungen der Formel (VII) in Betracht, welche bei Verfahren (f) als Reaktionskomponenten eingesetzt werden.

Verfahren (f) wird vorzugsweise in Gegenwart eines Katalysators durchgeführt. Als solche kommen vorzugsweise starke Säuren, wie z.B. Schwefelsäure, Salzsäure, p-Toluolsulfonsäure und Methansulfonsäure in Betracht.

Die Reaktionstemperaturen können bei dem erfindungsgemäßen Verfahren (f) in einem größeren Bereich variiert werden. Im allgemeinen arbeitet man bei Temperaturen zwischen 0 °C und 150 °C, vorzugsweise bei Temperaturen zwischen 20 °C und 100 °C.

Das erfindungsgemäße Verfahren (f) wird im allgemeinen unter Normaldruck durchgeführt. Es ist jedoch auch möglich, unter erhöhtem oder vermindertem Druck zu arbeiten.

Zur Durchführung von Verfahren (f) setzt man die Ausgangsverbindung der Formel (VII) im allgemeinen im Überschuß ein, so daß sie auch als Verdünnungsmittel dient.

Umsetzung und Aufarbeitung können nach üblichen Methoden durchgeführt werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich zur Bekämpfung von monokotylen und dikotylen Unkräutern, insbesondere im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.
Als feste Trägerstoffe kommen infrage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen,

Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Die Herstellung und die Verwendung der erfindungsgemäßen Wirkstoffe geht aus den nachfolgenden Beispielen hervor.

Herstellungsbeispiele:

Beispiel 1

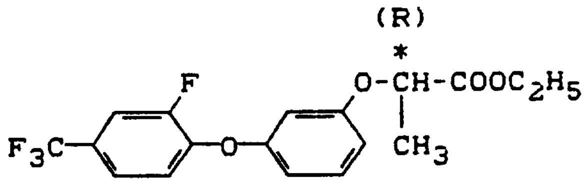

(Verfahren (a))

Eine Mischung aus 108 g (0,40 Mol) (S)-α-(4-Methyl-phenyl-sulfonyloxy)-propionsäure-ethylester, 120 g (0,40 Mol) 3-(2-Fluor-4-trifluormethyl-phenoxy)-phenol, 62 g (0,45 Mol) Kaliumcarbonat und 1 Liter Acetonitril wird 5 Stunden unter Rückfluß erhitzt. Nach Erkalten wird filtriert und vom Filtrat im Wasserstrahlvakuum das Lösungsmittel sorgfältig abdestilliert.

Man erhält 112 g (75% der Theorie) (R)-α-(3-(2-Fluor-4-trifluormethyl-phenoxy)-phenoxy)-propionsäure-ethylester als öligen Rückstand.
$n_D^{20}$ : 1,4972; Kp: 120 ° C/0,05 mbar.

Beispiel 2

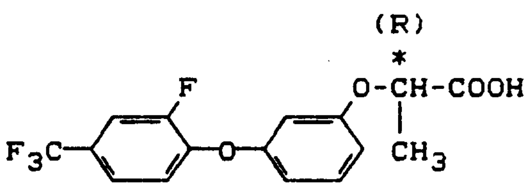

(Verfahren (c))

Eine Mischung aus 90 g (0,24 Mol) (R)-α-(3-(2-Fluor-4-trifluormethyl-phenoxy)-phenoxy)-propionsäure-ethylester, 250 ml Methanol, 107 ml Wasser und 36 ml konzentrierte Natronlauge (45%ig) wird 3 Stunden bei 50 ° C bis 60 ° C gerührt. Dann wird das Gemisch auf 2,5 Liter Wasser gegossen, mit konzentrierter Salzsäure auf pH1 angesäuert und mit Chloroform extrahiert. Die organische Phase wird mit Natriumsulfat getrocknet und filtriert. Vom Filtrat wird das Lösungsmittel im Wasserstrahlvakuum sorgfältig abdestilliert.

Man erhält 82 g (~100% der Theorie) (R)-α-(3-(2-Fluor-4-trifluormethyl-phenoxy)-phenoxy)-propionsäure als öligen Rückstand.
$n_D^{20}$ : 1,5015.

Beispiel 3

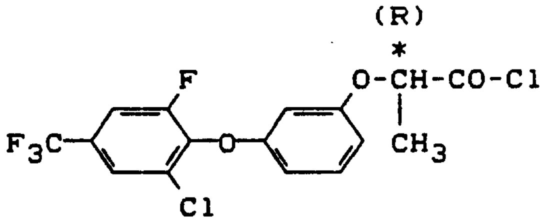

(Verfahren (d))

Eine Mischung aus 55 g (0,145 Mol) (R)-α-(3-(2-Chlor-6-fluor-4-trifluormethyl-phenoxy)-phenoxy)-propionsäure, 110 ml Methylenchlorid und 1 ml Dimethylformamid wird auf 45 ° C erhitzt und bei dieser Temperatur werden 21 g (0,174 Mol) Thionylchlorid tropfenweise dazugegeben. Das Reaktionsgemisch wird dann bis zum Ende der Gasentwicklung unter Rückfluß erhitzt und anschließend im Vakuum sorgfältig eingeengt.

Man erhält (R)-α-(3-(2-Chlor-6-fluor-4-trifluormethyl-phenoxy)-phenoxy)-propionsäurechlorid als öligen Rückstand, der ohne weitere Reinigung weiter verwendet wird (vgl. Beispiel 4).

Beispiel 4

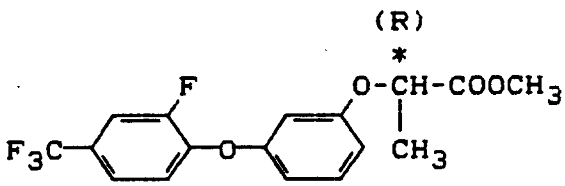

(Verfahren (e))

Eine Lösung von 69 g (R)-α-(3-(2-Fluor-4-trifluormethyl-phenoxy)-phenoxy)-propionsäurechlorid in 200 ml Toluol wird unter Rühren tropfenweise zu einer Mischung aus 9,6 g Methanol, 320 ml Toluol und 42 ml Pyridin bei 0 ° C bis 5 ° C gegeben. Nach einstündigem Rühren wird die Reaktionslösung mit 1N-Salzsäure, dann mit 1N-Natronlauge und schließlich mit Wasser gewaschen. Dann wird mit Natriumsulfat getrocknet und filtriert. Vom Filtrat wird das Lösungsmittel im Wasserstrahlvakuum sorgfältig abdestilliert.

Man erhält 60 g (83% der Theorie) (R)-α-(3-(2-Fluor-4-trifluormethyl-phenoxy)-phenoxy)-propionsäuremethylester als öligen Rückstand.

Kp: 120 ° C/0,1 mbar.

Analog zu den Herstellungsbeispielen 1 bis 4 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

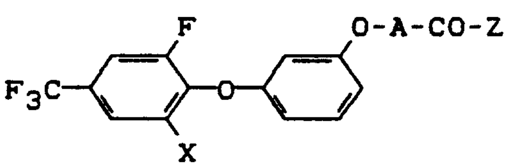

(I)

<u>Tabelle 1:</u> Beispiele für die Verbindungen der Formel (I)

| Bsp.-Nr. | A | X | Z | Physikalische Daten |
|---|---|---|---|---|
| 5 | -CH-<br>\|<br>$CH_3$ | Cl | $OC_2H_5$ | $n_D^{20}$ : 1,5049 |
| 6 | -CH-<br>\|<br>$CH_3$ | F | $OC_2H_5$ | $n_D^{20}$ : 1,4886 |
| 7 | -CH-<br>\|<br>$CH_3$ | Cl | OH | Fp.: 97° C |
| 8 | -CH-<br>\|<br>$CH_3$ | F | OH | Fp.: 125° C |
| 9 | -CH-<br>\|<br>$CH_3$ | F | Cl | (Öl) |

Tabelle 1 - Fortsetzung

| Bsp.-Nr. | A | X | Z | Physikalische Daten |
|---|---|---|---|---|
| 10 | $-CH(CH_3)-$ | H | Cl | (Öl) |
| 11 | $-CH(CH_3)-$ | Cl | $OCH_3$ | $n_D^{20}$ : 1,5083 |
| 12 | $-CH(CH_3)-$ | F | $OCH_3$ | $n_D^{20}$ : 1,4934 |
| 13 | $-CH(CH_3)-$ | Cl | $OCH(CH_3)_2$ | $n_D^{20}$ : 1,4975 |
| 14 | $-CH(CH_3)-$ | F | $OCH(CH_3)_2$ | $n_D^{20}$ : 1,4929 |
| 15 | $-CH(CH_3)-$ | H | $OCH(CH_3)_2$ | $n_D^{20}$ : 1,4926 |
| 16 | $-CH(CH_3)-$ | F | $OC_4H_9$ | $n_D^{20}$ : 1,4827 |
| 17 | $-CH(CH_3)-$ | Cl | $OC_4H_9$ | $n_D^{20}$ : 1,4981 |

Tabelle 1 - Fortsetzung

| Bsp.-Nr. | A | X | Z | Physikalische Daten |
|---|---|---|---|---|
| 18 | -CH-<br>$CH_3$ | F | $OCH_2CH(CH_3)_2$ | $n_D^{20}$ : 1,4921 |
| 19 | -CH-<br>$CH_3$ | H | $OC_4H_9$ | $n_D^{20}$ : 1,4920 |
| 20 | -CH-<br>$CH_3$ | Cl | $OCH_2CH(CH_3)_2$ | $n_D^{20}$ : 1,4970 |
| 21 | -CH-<br>$CH_3$ | H | $OCH_2CH(CH_3)_2$ | $n_D^{20}$ : 1,4913 |
| 22 | -CH-<br>$CH_3$ | H | $N(CH_3)_2$ | $n_D^{20}$ : 1,5181 |
| 23 | -CH-<br>$CH_3$ | H | $OCH_2COOC_2H_5$ | $n_D^{20}$ : 1,4988 |
| 24 | -CH-<br>$CH_3$ | H | $OCH_2Si(CH_3)_3$ | $n_D^{20}$ : 1,4905 |
| 25 | -CH-<br>$CH_3$ | H | $OCH_2CH_2SCH_2$- 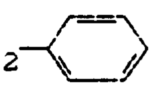 | $n_D^{20}$ : 1,5496 |
| 26 | -CH-<br>$CH_3$ | F | $OCH_2Si(CH_3)_3$ | $n_D^{20}$ : 1,4817 |

<u>Tabelle 1</u> - Fortsetzung

| Bsp.-Nr. | A | X | Z | Physikalische Daten |
|---|---|---|---|---|
| 27 | $-CH(CH_3)-$ | Cl | $OCH_2Si(CH_3)_3$ | $n_D^{20}$ : 1,4950 |
| 28 | $-CH(CH_3)-$ | F | $OCH_2CH_2SCH_2-$ 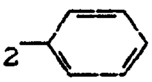 | $n_D^{20}$ : 1,5464 |
| 29 | $-CH(CH_3)-$ | F | $OCH_2COOC_2H_5$ | $n_D^{20}$ : 1,4887 |
| 30 | $-CH(CH_3)-$ | Cl | $OCH_2CH_2SCH_2-$ 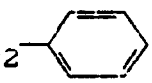 | $n_D^{20}$ : 1,5526 |
| 31 | $-CH(CH_3)-$ | Cl | $OCH_2C(CH_3)_3$ | $n_D^{20}$ : 1,4948 |
| 32 | $-CH(CH_3)-$ | H | $OCH_2C(CH_3)_3$ | $n_D^{20}$ : 1,4885 |
| 33 | $-CH(CH_3)-$ | F | $OCH_2C(CH_3)_3$ | $n_D^{20}$ : 1,4802 |
| 34 | $-CH(CH_3)-$ | F | $O(CH_2)_3OCH_2-$ 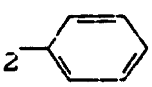 | $n_D^{20}$ : 1,5138 |

Tabelle 1 - Fortsetzung

| Bsp.-Nr. | A | X | Z | Physikalische Daten |
|---|---|---|---|---|
| 35 | $-CH-$<br>$CH_3$ | Cl | $O(CH_2)_3OCH_2$ 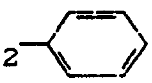 | $n_D^{20}$ : 1,5221 |
| 36 | $-CH-$<br>$CH_3$ | Cl | $N(CH_3)_2$ | Fp.: 95° C |
| 37 | $-CH-$<br>$CH_3$ | F | $N(CH_3)_2$ | Fp.: 80° C |
| 38 | $-CH-$<br>$CH_3$ | H | $OCHCOOCH_3$<br>$CH_3$ | $n_D^{20}$ : 1,4972 |
| 39 | $-CH-$<br>$CH_3$ | F | $OCHCOOCH_3$<br>$CH_3$ | $n_D^{20}$ : 1,4891 |
| 40 | $-CH-$<br>$CH_3$ | Cl | $OCHCOOCH_3$<br>$CH_3$ | $n_D^{20}$ : 1,5010 |
| 41 | $-CH-$<br>$CH_3$ | H | $OCH_2CH_2OCH_2CH_2OCH_3$ | $n_D^{20}$ : 1,4975 |
| 42 | $-CH-$<br>$CH_3$ | F | $OCH_2$ 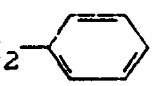 | $n_D^{20}$ : 1,5212 |
| 43 | $-CH-$<br>$CH_3$ | Cl | $OCH_2$ 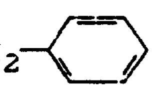 | $n_D^{20}$ : 1,5344 |

<u>Tabelle 1</u> - Fortsetzung

| Bsp.-Nr. | A | X | Z | Physikalische Daten |
|---|---|---|---|---|
| 44 | -CH-<br>\|<br>$CH_3$ | H | $OCH_2$ 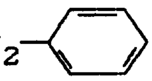 | $n_D^{20}$ : 1,5312 |
| 45 | -CH-<br>\|<br>$CH_3$ | H | $OCH_2CH_2SC_2H_5$ | $n_D^{20}$ : 1,5140 |
| 46 | -CH-<br>\|<br>$CH_3$ | H | $NHCH_2COOC_2H_5$ | Fp.: 87° C |
| 47 | -CH-<br>\|<br>$CH_3$ | H | $OCH_2CON(C_2H_5)_2$ | $n_D^{20}$ : 1,5110 |
| 48 | -CH-<br>\|<br>$CH_3$ | H | $O(CH_2)_3OCH_2$ 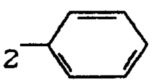 | $n_D^{20}$ : 1,5185 |
| 49 | -CH-<br>\|<br>$CH_3$ | H | $OCH_2C-N(CH_3)_2$<br>‖<br>O | $n_D^{20}$ : 1,5155 |
| 50 | -CH-<br>\|<br>$CH_3$ | H | $OCH_2$ 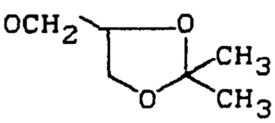 | $n_D^{20}$ : 1,4996 |

Tabelle 1 - Fortsetzung

| Bsp.-Nr. | A | X | Z | Physikalische Daten |
|---|---|---|---|---|
| 51 | -CH-<br>$CH_3$ | H | 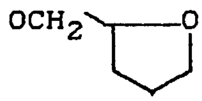 | $n_D^{20}$ : 1.5083 |
| 52 | -CH-<br>$CH_3$ | Cl | $OCH_2COOC_2H_5$ | $n_D^{20}$ : 1.4886 |
| 53 | -CH-<br>$CH_3$ | Cl | $OCH_2CH_2CH_2CH_3$ | $n_D^{20}$ : 1.4976 |
| 54 | -CH-<br>$CH_3$ | Cl | $OCH_2CH_2CH_3$ | $n_D^{20}$ : 1.5005 |
| 55 | -CH-<br>$CH_3$ | Cl | $O(CH_2)_5CH_3$ | $n_D^{20}$ : 1.4919 |
| 56 | -CH-<br>$CH_3$ | Cl | 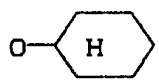 | $n_D^{20}$ : 1.4932 |
| 57 | -CH-<br>$CH_3$ | F | $OCH_2CH_2CH_3$ | $n_D^{20}$ : 1.4844 |
| 58 | -CH-<br>$CH_3$ | F | $O(CH_2)_4CH_3$ | $n_D^{20}$ : 1.4789 |
| 59 | -CH-<br>$CH_3$ | F | $O(CH_2)_5CH_3$ | $n_D^{20}$ : 1.4750 |
| 60 | -CH-<br>$CH_3$ | F | 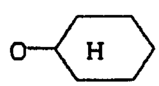 | $n_D^{20}$ : 1.4886 |

Anwendungsbeispiele:

In den folgenden Anwendungsbeispielen wird die nachstehend aufgeführte Verbindung als Vergleichssubstanz herangezogen:

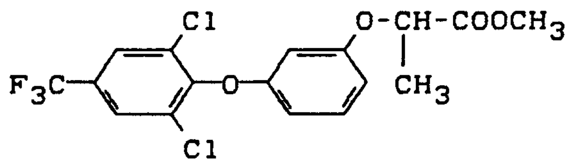

(A)

α-(3-(2,6-Dichlor-4-trifluormethyl-phenoxy)-phenoxy)-propionsäure-methylester (bekannt aus DE-OS 2805981, Bsp. 1)

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

Eine deutliche Überlegenheit in der Wirksamkeit gegenüber dem Stand der Technik zeigen in diesem Test z.B. die Verbindungen gemäß folgender Herstellungsbeispiele: 5 und 6.

**Patentansprüche**

**1.** Phenoxyphenoxycarbonsäurederivate der Formel (I)

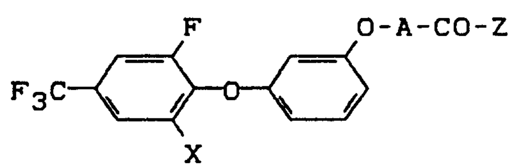

(I)

in welcher

A für Alkandiyl mit 1 bis 3 Kohlenstoffatomen steht,

X für Wasserstoff oder Halogen steht und

Z für Chlor, Hydroxy, Amino, $C_1$-$C_6$-Alkylamino, $C_3$-$C_4$-Alkenylamino, $C_3$-$C_4$-Alkinylamino, Phenylamino, Benzylamino, $C_1$-$C_4$-Alkoxycarbonyl-$C_1$-$C_2$-alkylamino, Cyanamino, Di-($C_1$-$C_4$-alkyl)-amino, Di-($C_3$-$C_4$-alkenyl)-amino, $C_1$-$C_4$-Alkylsulfonylamino, Phenylsulfonylamino, Tolylsulfonylamino, Hydroxyamino, $C_1$-$C_6$-Alkoxyamino, N-($C_1$-$C_6$-Alkoxy)-N-($C_1$-$C_4$-alkyl)-amino, Hydrazino, $C_1$-$C_4$-Alkylsulfonylhydrazino, Phenylsulfonylhydrazino, Tolylsulfonylhydrazino, $C_1$-$C_4$-Alkylthio, Phenylthio, Benzylthio, $C_1$-$C_4$-Alkoxy-carbonyl-$C_1$-$C_2$-alkylthio oder für die Gruppierung -O-$R^1$ steht, worin

$R^1$ für einen gegebenenfalls durch Fluor und/oder Chlor substituierten Rest aus der Reihe $C_1$-$C_6$-Alkyl, $C_3$-$C_4$-Alkenyl, $C_3$-$C_4$-Alkinyl, $C_3$-$C_6$-Cycloalkyl, $C_1$-$C_4$-Alkoxy-$C_1$-$C_4$-alkyl, $C_1$-$C_4$-Alkoxy-$C_1$-$C_4$-Alkoxy-$C_1$-$C_4$-alkyl, $C_1$-$C_4$-Alkylthio-$C_1$-$C_4$-alkyl, $C_1$-$C_4$-Alkylsulfinyl-$C_1$-$C_4$-alkyl, $C_1$-$C_4$-Alkylsulfonyl-$C_1$-$C_4$-alkyl, Phenoxy-$C_1$-$C_3$-alkyl, Trimethylsilylmethyl, Phenylthio-$C_1$-$C_3$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-(Di)-Alkylamino-carbonyl-$C_1$-$C_2$-alkyl, Benzyl, Pyrazolyl-$C_1$-$C_4$-alkyl, $C_2$-$C_4$-Alkylidenamino oder für ein Ammonium-, ein $C_1$-$C_4$-Alkylammonium ein Natrium-, Kalium- oder Calcium-äquivalent steht, oder
die Gruppierung

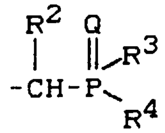

steht, worin

$R^2$ für Wasserstoff, $C_1$-$C_4$-Alkyl, Phenyl, Furyl, Thienyl oder Pyridyl steht,
$R^3$ für $C_1$-$C_4$-Alkyl oder $C_1$-$C_4$-Alkoxy steht,
$R^4$ für $C_1$-$C_4$-Alkoxy steht und
Q für Sauerstoff oder Schwefel steht, oder
$R^1$ für die Gruppierung $-(CH_2)_n-R^5$ steht, worin
n für die Zahlen 0, 1 oder 2 steht und
$R^5$ für einen gegebenenfalls durch Fluor, Chlor, Brom und/oder $C_1$-$C_4$-Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Isoxazolidinyl, Pyridinyl oder Pyrimidinyl steht.

**2.** Verbindungen der Formel (I), gemäß Anspruch 1, in welcher
A für Methan-1,1-diyl (Methylen, $-CH_2-$), Ethan-1,1-diyl

(Ethyliden, 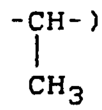),

Ethan-1,2-diyl (Ethylen, Dimethylen, $-CH_2CH_2-$), Propan-1,1-diyl

(Propyliden, 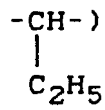),

X für Wasserstoff, Fluor oder Chlor steht und
Z für Chlor, Hydroxy, Amino, $C_1$-$C_4$-Alkylamino, Phenylamino, $C_1$-$C_4$-Alkoxy-carbonyl-$C_1$-$C_2$-alkylamino, Di-($C_1$-$C_3$-alkyl)-amino, $C_1$-$C_4$-Alkylsulfonylamino, Phenylsulfonylamino, Hydroxyamino, Cyanamino, $C_1$-$C_4$-Alkoxyamino, N-($C_1$-$C_4$-Alkoxy)-N-($C_1$-$C_3$-alkyl)-amino, Hydrazino, $C_1$-$C_4$-Alkylsulfonylhydrazino, Phenylsulfonylhydrazino, $C_1$-$C_4$-Alkylthio oder $C_1$-$C_4$-Alkoxy-carbonyl-$C_1$-$C_2$-alkylthio oder für die Gruppierung $-O-R^1$ steht, worin
$R^1$ für $C_1$-$C_4$-Alkyl, $C_1$-$C_2$-Alkoxy-$C_1$-$C_2$-alkyl, $C_1$-$C_2$-Alkoxy-$C_1$-$C_2$-alkoxy-$C_1$-$C_2$-alkyl, $C_1$-$C_2$-Alkylthio-$C_1$-$C_2$-alkyl, $C_1$-$C_2$-Alkylsulfinyl-$C_1$-$C_2$-alkyl, $C_1$-$C_2$-Alkylsulfonyl-$C_1$-$C_2$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-(Di)-Alkylamino-carbonyl-$C_1$-$C_2$-alkyl, Benzyl, Trimethylsilylmethyl oder für ein Ammonium-, $C_1$-$C_3$-Alkylammonium-, Natrium-, oder Kalium-äquivalent steht, oder für die Gruppierung

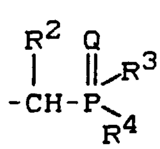

steht, worin

$R^2$ für Wasserstoff, Methyl, Phenyl, Furyl, Thienyl oder Pyridyl steht,

$R^3$ für Methoxy oder Ethoxy steht,
$R^4$ für Methoxy oder Ethoxy steht und
Q für Sauerstoff oder Schwefel steht, oder
$R^1$ für die Gruppierung $-(CH_2)_n-R^5$ steht, worin
n für die Zahlen 0, 1 oder 2 steht und
$R^5$ für einen gegebenenfalls durch Chlor und/oder Methyl substituierten heterocyclischen Rest aus der Reihe Furyl, Tetrahydrofuryl, Thienyl, Perhydropyranyl, Oxazolyl, Thiazolyl, Isoxazolidinyl und Dioxolanyl steht.

**3.** Verbindungen der Formel (I), gemäß Anspruch 1, in welcher
A für Ethan-1,1-diyl

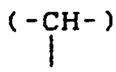

steht,
X für Wasserstoff, Fluor oder Chlor steht und
Z die oben als bevorzugt angegebene Bedeutung hat.

**4.** Verbindungen der Formel (I), gemäß Anspruch 1, in welcher die Verbindungen der Formel (I) in Form der R-Isomeren vorliegen.

**5.** Herbizide Mittel, gekennzeichnet durch einen Gehalt an mindestens einem Phenoxyphenoxycarbonsäurederivat der Formel (I) gemäß Anspruch 1.

**6.** Verfahren zur Bekämpfung von Unkräutern, dadurch gekennzeichnet, daß man Phenoxyphenoxycarbonsäurederivate der Formel (I) gemäß Anspruch 1 auf die Unkräuter und/oder ihren Lebensraum einwirken läßt.

**7.** Verfahren zur Herstellung von Phenoxyphenoxycarbonsäurederivaten der Formel (I), dadurch gekennzeichnet, daß man
(a) fluorierte Phenoxyphenole der allgemeinen Formel (II)

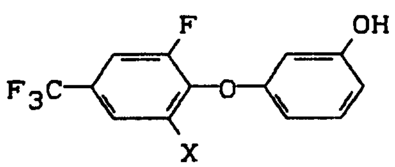

(II)

in welcher
X die in Anspruch 1 angegebene Bedeutung hat,
mit Carbonsäurederivaten der allgemeinen Formel (III)

Y-A-CO-Z (III)

in welcher
A und Z die in Anspruch 1 angegebene Bedeutung haben und
Y für eine nucleophile Abgangsgruppe steht,
und gegebenenfalls in Gegenwart eines Verdünnungsmittels umsetzt, oder
(b) Fluorbenzolderivate der allgemeinen Formel (IV)

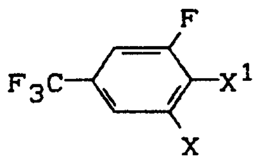

(IV)

in welcher

X die oben angegebene Bedeutung hat und

$X^1$ für Halogen steht,

mit Hydroxyphenoxycarbonsäurederivaten der allgemeinen Formel (V)

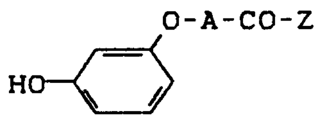

(V)

in welcher

A und Z die oben angegebene Bedeutung haben,

gegebenenfalls in Gegenwart eines Säureakzeptors und gegebenenfalls in Gegenwart eines Verdünnungsmittels umsetzt, oder

(c) für den Fall, daß Z für Hydroxy steht und A und X die oben angegebene Bedeutung haben,

Verbindungen der Formel (I), in welcher Z für Methoxy oder Ethoxy steht und A und X die oben angegebene Bedeutung haben,

mit einem Alkalihydroxid in Gegenwart von Wasser und gegebenenfalls in Gegenwart eines organischen Lösungsmittels umsetzt und dann - gegebenenfalls nach Einengen - mit einer Mineralsäure ansäuert, oder

(d) für den Fall, daß Z für Chlor steht und A und X die oben angegebene Bedeutung haben

Verbindungen der Formel (I), in welcher Z für Hydroxy steht und A und X die oben angegebene Bedeutung haben,

mit einem Chlorierungsmittel gegebenenfalls in Gegenwart eines Katalysators und gegebenenfalls in Gegenwart eines Verdünnungsmittels umsetzt, oder

(e) für den Fall, daß Z mit Ausnahme von Chlor die oben angegebene Bedeutung hat und A und X die oben angegebene Bedeutung haben,

Verbindungen der Formel (I), in welcher Z für Chlor steht und A und X die oben angegebene Bedeutung haben, mit Verbindungen der allgemeinen Formel (VI)

H-Z (VI)

in welcher

Z mit Ausnahme von Chlor die oben angegebene Bedeutung hat,

gegebenenfalls in Gegenwart eines Säureakzeptors und gegebenenfalls in Gegenwart eines Verdünnungsmittels umsetzt, oder

(f) für den Fall, daß Z für die Gruppierung $-O-R^1$ steht, worin $R^1$ mit Ausnahme von Ammonium, Alkylammonium, Alkalimetall und Erdalkalimetall die oben angegebene Bedeutung hat und A und X die oben angegebene Bedeutung haben,

Verbindungen der Formel (I), in welcher Z für Hydroxy, Methoxy oder Ethoxy steht und A und X die oben angegebene Bedeutung haben, mit Hydroxyverbindungen der allgemeinen Formel (VII)

$HO-R^1$ (VII)

in welcher

$R^1$ mit Ausnahme von Ammonium, Alkylammonium, Alkalimetall und Erdalkalimetall die oben angegebene Bedeutung hat,

gegebenenfalls in Gegenwart eines Katalysators und gegebenenfalls in Gegenwart eines Verdünnungsmittels umsetzt.

8. Verwendung von Phenoxyphenoxycarbonsäurederivaten der Formel (I) gemäß den Ansprüchen 1 bis 4 zur Bekämpfung von Unkräutern.

9. Verfahren zur Herstellung von herbiziden Mitteln, dadurch gekennzeichnet, daß man Phenoxyphenoxycarbonsäurederivate der Formel (I) gemäß den Ansprüchen 1 bis 4 mit Streckmitteln und/oder oberflächlichen Substanzen vermischt.

Europäisches Patentamt

# EUROPÄISCHER RECHERCHENBERICHT

Nummer der Anmeldung

EP 92 11 6537

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-A-2 639 796 (ROHM & HAAS CO.)<br>* Ansprüche 1-5,14,15,33-36,40,41 *<br>--- | 1-3,5-8 | C07C59/68 |
| X | DE-A-2 716 189 (SOCIETE DE RECHERCHES INDUSTRIELLES S.O.R.I. S.A.)<br>* Ansprüche 1,3-5,15 *<br>--- | 1-3,5-8 | |
| X | DE-A-2 732 442 (CIBA-GEIGY AG)<br>* Ansprüche 1,2,5-8 *<br>--- | 1-3,5-8 | |
| D,X | EP-A-0 127 808 (BAYER AG)<br>* Seite 7, Zeile 16 - Seite 13, Zeile 7 *<br>* Seite 23, Zeile 9 - Seite 24, Zeile 3 *<br>* Ansprüche 1-7 *<br>--- | 1-8 | |
| D,X | DE-A-3 425 123 (BAYER AG)<br>* Ansprüche 1-7 *<br>--- | 1-8 | |
| D,X | EP-A-0 370 644 (I.C.I.)<br>* Seite 3, Zeile 4 - Zeile 5 *<br>* Seite 16, Zeile 8 - Zeile 58 *<br>* Ansprüche 1,7,8 *<br>----- | 1-9 | RECHERCHIERTE SACHGEBIETE (Int. Cl.5)<br>C07C |

Der vorliegende Recherchenbericht wurde für alle Patentansprüche erstellt

| Recherchenort | Abschlußdatum der Recherche | Prüfer |
|---|---|---|
| DEN HAAG | 11 JANUAR 1993 | KLAG M.J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)